(19) Europäisches Patentamt
European Patent Office
Office européen des brevets

(11) Publication number: **0 094 443**
**A1**

(12) # EUROPEAN PATENT APPLICATION

(21) Application number: 82104290.0

(22) Date of filing: 17.05.82

(51) Int. Cl.³: C 07 D 309/30
C 07 C 69/30, C 07 C 69/33
A 61 K 31/22, A 61 K 31/365

(43) Date of publication of application: 23.11.83 Bulletin 83/47

(84) Designated Contracting States: AT BE CH DE FR GB IT LI LU NL SE

(71) Applicant: MERCK & CO. INC.
126, East Lincoln Avenue P.O. Box 2000
Rahway New Jersey 07065(US)

(72) Inventor: Willard, Alvin K.
8 Maplewood Lane
Wilmington Delaware 19810(US)

(72) Inventor: Hoffman, William F.
740 Weikel Road
Lansdale Pennsylvania 19446(US)

(72) Inventor: Smith, Robert L.
1355 Pickwick Lane
Lansdale Pennsylvania 19446(US)

(74) Representative: Abitz, Walter, Dr.-Ing. et al,
Abitz, Morf, Gritschneder, Freiherr von Wittgenstein
Postfach 86 01 09
D-8000 München 86(DE)

(54) **6(R)-[2-[8(S)(2,2-dimethylbutyryloxy)-2(S),6(S)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one, process for preparing and pharmaceutical composition containing the same.**

(57) 6(R)-[2-[8(S)(2,2-dimethylbutyryloxy)-2(S),6(S)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one, a derivative of mevinolin is produced by reduction of mevinolin's allylic double bond system, saponification of the 2(S)-methylbutyryl ester, protection of the 4(R)-hydroxy, not necessarily in that order, followed by esterification of the 8(S)-hydroxy with a 2,2-dimethylbutyryl group and deprotection of the masked 4(R)-hydroxy group. The resulting compound is a potent antihypercholesterolemic agent.

EP 0 094 443 A1

TITLE OF THE INVENTION

6(R)-[2-[8(S)(2,2-dimethylbutyryloxy)-2(S),6(S)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one, process for preparing and pharmaceutical composition containing the same

BACKGROUND OF THE INVENTION

This invention is concerned with the compound of structural formula I or Ia:

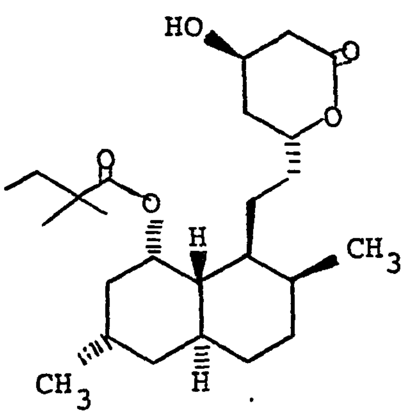

I

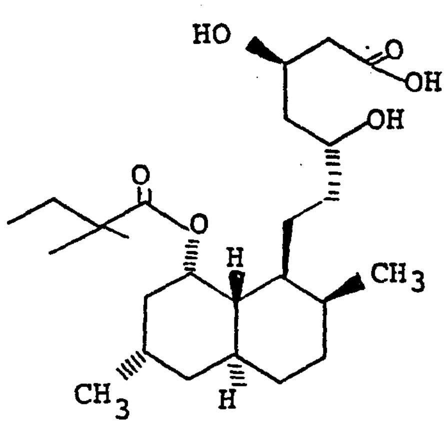

Ia

or a pharmaceutically acceptable salt of Ia such as the sodium, potassium, calcium, magnesium, ammonium salt or the like or an alkyl ester such as the methyl, ethyl or α-monoglyceride, antihyper-chloesterolemic agents, and with processes for their preparation.

A few compounds have been reported in the literature with structures and biological activities similar to Compound I: Endo, in U.S. Patents 3,983,140 described fermentative production of the compound that has become known as ML-236B and compactin; Monaghan et al. in U.S. Patent 4,231,938 and Albers-Schonberg et al. in U.S. Patent 4,294,846 described the fermentative production, with another organism, of mevinolin and 4a, 5,-dihydromevinolin respectively; and the published Japanese application (Kokai) 55009-024 describes a tetrahydro compactin wherein the polyhydronaphthyl moiety becomes a decalin.

Now with the present invention there is provided a new compound of formula I derived from mevinolin, having a trans-decalin moiety in place of the hexahydronaphthyl group and an 8(S)-(2,2-dimethyl-butyryloxy) group instead of the natural 8(S)-[2(S)-methylbutyryl]group.

There are also provided processes for the preparation of the novel compound of this invention.

## DETAILED DESCRIPTION OF THE INVENTION

The novel compound of this invention is the compound of structural formula I or Ia:

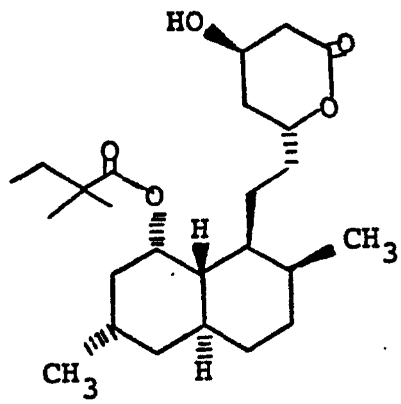

I

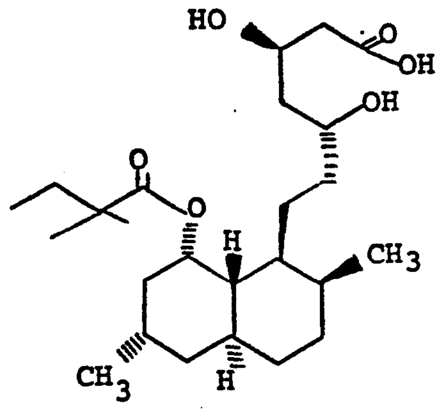

Ia .

or a pharmaceutically acceptable salt of Ia such as the sodium, potassium, calcium, magnesium, ammonium salt or the like, or an alkyl ester such as the methyl, ethyl, or α-monoglyceride.

The processes of this invention are depicted by the following synthetic Schemes I and II.

Synthetic Scheme I

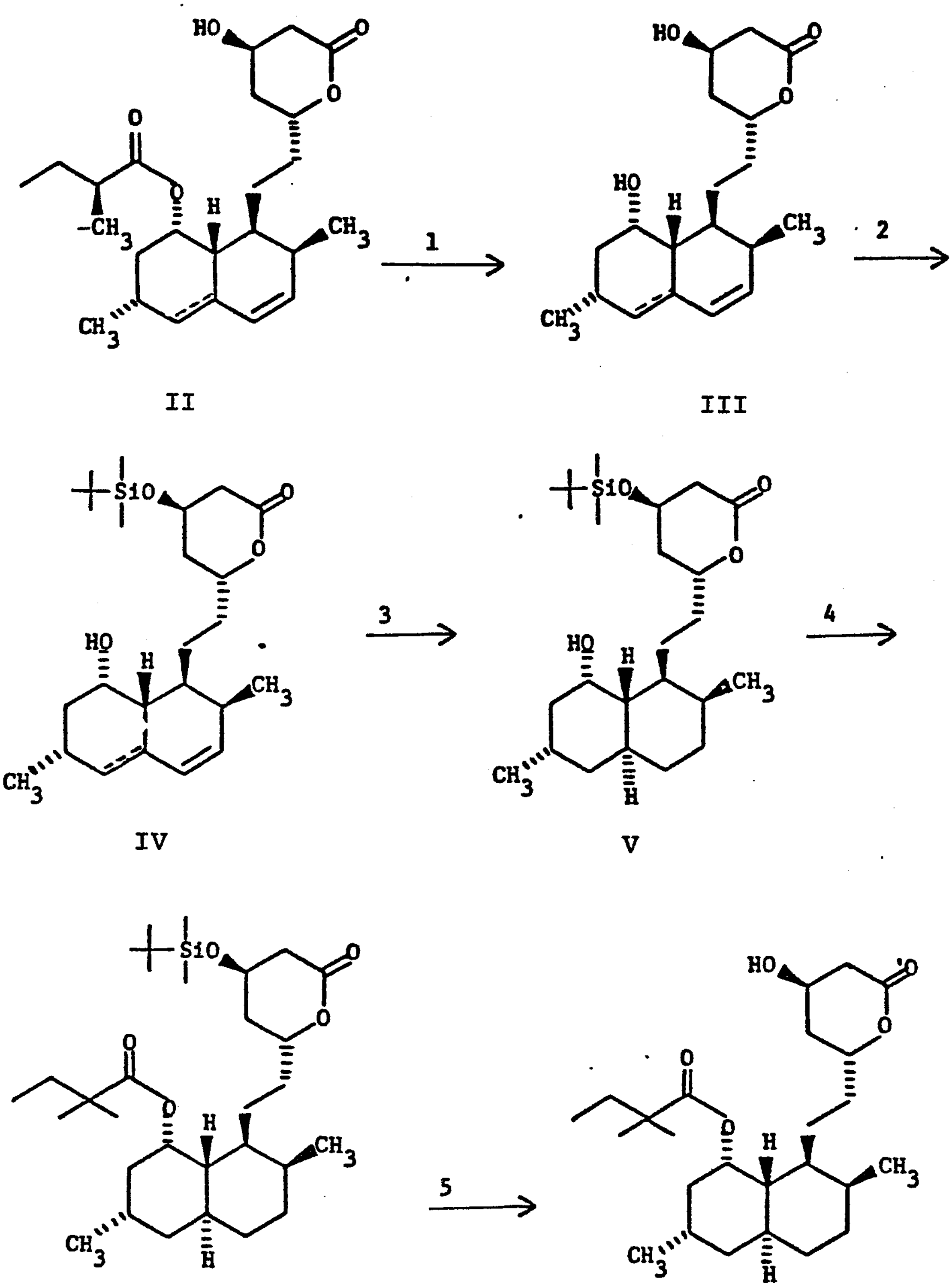


II III

IV V

VI I

Synthetic Scheme II

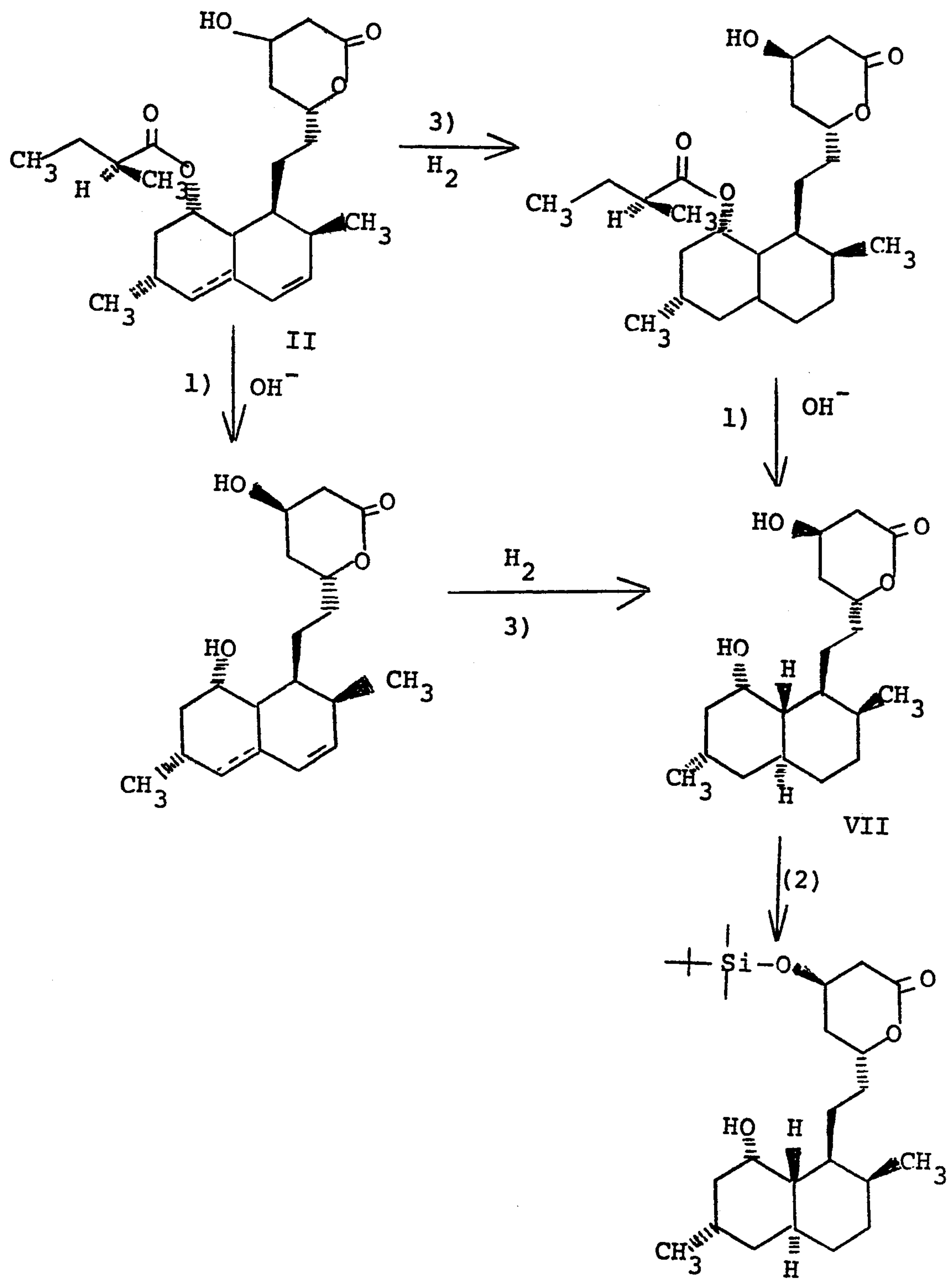


V

The Synthetic Schemes I and II illustrate 3 different routes from the identical starting material to arrive at intermediate compound V, following which there are 2 steps to the novel compound I of this invention. The processes of these steps comprise the following:

Step 1: The compound MK-803 (mevinolin) or the 4a, 5 dihydro derivative thereof is treated with an excess of lithium hydroxide in aqueous solution at 50-100° (reflux temperature) for 2 to about 4 days, cooled, acidified, extracted with a solvent and heated in toluene solution to effect lactonization.

Step 2: The diol III or VII is mono-silylated by treatment wit *tert*-butyldimethylsilyl chloride and imidazole in an aprotic solvent such as DMF at or about ambient temperature for 12 to about 24 hours to provide the 4-silyl ether.

Step 3: The double bonds of the polyhydronaphthyl moiety are reduced with hydrogen and a precious metal catalyst such as platinum oxide in an organic solvent such as ethyl acetate under a pressure of 1 to 100 atmospheres at ambient temperature for about 10 to about 20 hours. After isolation of the resulting decalin the product is chromatographed, preferably on silica gel from which only the *trans*-decalin V is isolated.

Step 4: The 8-hydroxyl group of V is esterified by treatment with 2,2-dimethylbutyryl chloride in pyridine with or without an acylation catalyst such as 4-pyrrolidinopyridine, 4-dimethylaminopyridine, or

4-hydroxybenztriazole at about 100 to about 140° for 4 to about 10 hours.

Step 5: The 4-O-silyl group is removed by treatment of compound VI with about 4 equivalents of glacial acetic acid and about 3 equivalents of tetrabutylammonium fluoride trihydrate in an ethereal solvent such as THF, ether, 1,2-dimethoxyethane or the like in an inert atmosphere at about 15° to about 30° for 1 to 3 days.

The compound of this invention is useful as an antihypercholesterolemic agent for the treatment of atherosclerosis, hyperlipemia and like diseases. It may be administered orally or parenterally in the form of a capsule, a tablet, an injectable preparation or the like. It is usually desirable to use the oral route. Doses may be varied, depending on the age, severity, body weight and other conditions of the patients but daily dosage for adult humans is within a range of from about 2 mg to 2000 mg (preferably 10 to 100 mg) given in three or four divided doses. Higher doses may be favorably applied as required.

The compound of this invention also has useful anti-fungal activities. For example, it may be used to control strains of *Penicillium sp.*, *Aspergillus niger*, *Cladosporium sp.*, *Cochliobolus miyabeanus* and *Helminthosporium cynodnotis*. For those utilities it is admixed with suitable formulating agents, powders, emulsifying agents or solvents such as aqueous ethanol and sprayed or dusted on the plants to be protected.

## EXAMPLE 1

Step 1: Preparation of 6(R)-[2-(8(S)-Hydroxy-2(S), 6(R)-dimethyl-1,2,6,7,8,8a(R)-hexahydro-naphthyl-1(S)ethyl]-4(R)-hydroxy-3,4,5,6-tetrahydro-2H-pyran-2-one (2).

A mixture of mevinolin (50.2 g, 0.124 mole) and $LiOH.H_2O$ (52.0 g, 1.24 mole) in water (3 L) was magnetically stirred at reflux under a $N_2$ atmosphere for 72 hours. The stirred mixture was cooled to 0°C (ice/acetone bath) and treated with 12N HCl (120 ml, 1.44 mole) at such a rate that the temperature did not exceed 3°C. This mixture was saturated with solid NaCl and extracted with ether (4 x 500 ml). The combined extracts were washed with brine (2 x 250 ml), dried over $MgSO_4$ and evaporated to give an orange oil (31.7 g). This oil was dissolved in toluene (250 ml) and the solution was refluxed under a $N_2$ atmosphere for 4 hours with continuous separation of the water in a Dean-Stark apparatus to effect relactonization. Evaporation of the toluene left an oily residue which was dissolved in ether (1.5 L). This solution was washed with saturated $NaHCO_3$ (250 ml), $H_2O$ (250 ml) and brine (250 ml), dried and evaporated to provide a solid residue. Trituration of this solid with hexane (200 ml) gave the title compound as a cream colored solid (29.7 g, 75%) which did not require further purification for synthetic purposes. An analytical

sample was prepared by recrystallizing a portion of the above solid from n-butyl chloride to provide colorless clusters, mp 128-131°C.

Step 2: Preparation of 6(R)-[2-(8(S)-Hydroxy-2(S), 6(R)-dimethyl-1,2,6,7,8,8a(R)-hexahydro-naphthyl-1(S))ethyl]-4(R)-tert-butyldimethyl-silyloxy-3,4,5,6-tetrahydro-2H-pyran-2-one(3)

A solution of the alcohol III (18.3 g, 0.057 mole), tert-butyldimethylsilylchloride (10.3 g, 0.068 mole) and imidazole (9.3 g, 0.137 mole) in DMF (200 ml) was magnetically stirred at ambient temperature for 18 hours. The reaction mixture was diluted with ether (1500 ml) and washed successively with water (200 ml), 2% aqueous HCl (200 ml), water (200 ml), saturated $NaHCO_3$ (200 ml) and water (2 x 200 ml) and dried over $MgSO_4$. The filtered ether solution was concentrated to one liter, diluted with 600 ml hexane and the resultant solution concentrated to 600 ml to provide a white solid (13.7 g). A second crop of crystals (3.4 g) was obtained by reducing the mother liquor to 250 ml and storing at 0°C overnight. The combined yield was 17.1 g (69%) and the solid melted at 142-4°C.

Step 3: Preparation of 6(R)-[2-(8(S)-Hydroxy-2(S), 6(S)-dimethyl-1,2,3,4,4a(S),-5,6,7,8,8a(S)-decahydronaphthyl-1(S)ethyl]-4(R)-tert-butyl-dimethylsilyloxy-3,4,5,6-tetrahydro-2H-pyran-2-one (4)

A mixture of the silyl ether IV (5.0 g, 0.0115 mole), and $PtO_2$ (1.0 g) in ethylacetate (200 ml) was hydrogenated in the Paar low pressure hydrogenator overnight. The catalyst was removed by filtration and the filtrate was concentrated to dryness leaving a white solid. This solid was chromatographed on a 80 mm column containing 7" of silica gel (230-400 mesh). Elution under air pressure with methylene chloride/acetone (98:2, v:v, 1.5 L) provided a forerun which was discarded. Continued elution with the same eluant (2 L) gave the title compound as a white solid (3.4 g, 67%), mp 146-7°C.

Step 4: Preparation of 6(R)-[2-[8(S)-(2,2-Dimethylbutyryloxy)-2(S),6(S)-dimethyl-1,2,3,4,4a(S), 5,6,7,8,8a(S)-decahydronaphthyl-1(S)]ethyl]-4(R)-tert-butyl dimethylsilyloxy-3,4,5,6-tetrahydro-2H-pyran-2-one (5)

The 2,2-dimethylbutyryl chloride (0.067 g, 0.0005 mole) was added to a magnetically stirred solution of the alcohol V (0.11 g, 0.00025 mole) and 4-pyrrolidinopyridine (0.0074 g, 0.00005 mole) in

pyridine (2 ml). After heating this solution at 100°C for 3 hours under $N_2$, another 0.0335 g of 2,2-dimethylbutyryl chloride and 0.0039 g of 4-pyrrolidinopyridine was added and heating was continued for another 3 hours.

The reaction was cooled, diluted with ether (50 ml), and washed with 3N HCl (2 x 5 ml) and brine (2 x 10 ml). The ether solution was dried over $MgSO_4$, filtered and evaporated to provide a yellow oil (0.136 g). This oil was chromatographed on a 30 mm column containing 6" of silica gel (230-400 mesh). The column was eluted under air pressure with $CH_2Cl_2$ (300 ml) and then $CH_2Cl_2$/acetone (98:2, v:v, 100 ml) to provide a forerun which was discarded. Continued elution with $CH_2Cl_2$/acetone (98:2, v:v, 50 ml) gave the desired product as a pale yellow oil (0.081 g, 60%).

Step 5: Preparation of 6(R)-[2-[8(S)-(2,2-Dimethylbutyryloxy)-2(S)-6(S)-dimethyl-1,2,3,4,4a(S),5,6,7,8,8a(S)-decahydronaphthyl-1(S)]ethyl]-4(R)-hydroxy-3,4,5,6-tetrahydro-2H-pyran-2-one

The silyl ether VI (0.5 g, 0.00093 mole) was added to a THF (30 ml) solution containing glacial HOAc (0.224 g, 0.00372 mole) and tetrabutylammonium fluoride trihydrate (0.88 g, 0.00279 mole). This solution was stirred magnetically under a $N_2$ atmosphere for 48 hours. The reaction solution was diluted with ether (150 ml) and washed with $H_2O$ (25

ml) and brine (2 x 75 ml). The ether solution ws dried over $MgSO_4$, filtered and evaporated to give a viscous oil (0.45 g). This oil was chromatographed on a 40 mm column containing 6" of silica gel (230-400 mesh). The column was eluted with $CH_2Cl_2$/acetone (85:15, v:v) under air pressure and 20 ml fractions were collected. Fractions 21-37 were combined and concentrated to dryness to provide the title compound as a solid (0.37 g, 94%). An analytical sample was prepared by recrystallizing the solid from ether/hexane to give colorless plates, mp 159-160°C.

EXAMPLE 2

Ethyl 3(R),5(R)-dihydroxy-7-[8(S)-(2,2-dimethylbutyryloxy)-2(S), 6(S)-dimethyl-1,2,3,4,4a(S), 5,6,7,8,8a(S)-decahydronaphthyl-1(S)]heptanoate

Sodium methoxide (30 mg) is added to a stirred suspension of Compound I (3.0 g) in ethanol (50 ml) under a nitrogen atmosphere. The resultant solution is stirred at ambient temperature for 1/2 hour and then diluted with ether (300 ml). The ethereal solution is washed with $H_2O$ (3 x 50 ml), dried over $MgSO_4$ and filtered. The filtrate is evaporated *in vacuo* leaving an oil which is chromatographed on a 60 mm column containing 6" of silica gel (230-400 mesh). Elution with methylene chloride/ethanol (96:4, V:V, 250 ml) under air pressure gives the title compound as a solid.

EXAMPLE 3

2,3-Dihydroxypropyl 3(R),5(R)-dihydroxy-7-[8(S)-(2,2-dimethylbutyryloxy)-2(S),6(S)-dimethyl-1,2,3,4,4a(S),5,6,7,8,8a(S)-decahydronaphthyl-1(S)] heptanoate

The sodium salt of compound Ia is prepared by adding 1N NaOH (.55 ml) to a solution of Compound I, 0.22 g in DMF (2 ml). After stirring this solution for 15 minutes, 1-iodo-2,3-dihydroxypropane (0.2 g) is added and the stirred solution is heated at 80°C (oil bath) for 6 hours. After cooling to ambient temperature, the reaction solution is poured into ether (100 ml). This ethereal solution is washed with brine (2 x 25 ml), dried over $MgSO_4$ and filtered. The filtrate is evaporated *in vacuo* leaving an oil which is chromatographed on a 20 mm column containing 6" of silica gel (230-400 mesh). Elution with acetone-methylene chloride (60:40; v:v;) under air pressure provides the title compound as an oil which solidifies upon storing in the freezer overnight.

WHAT IS CLAIMED IS:

1. A process for the preparation of a compound of structural formula I or Ia:

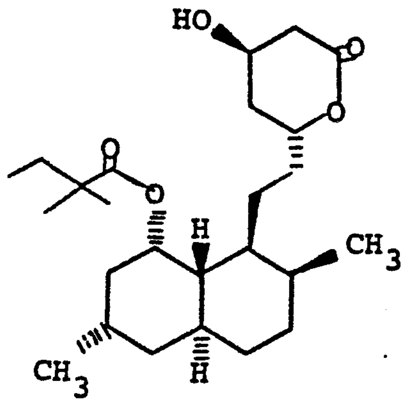

I

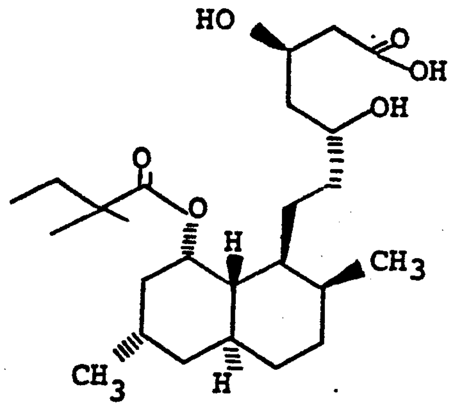

Ia .

or a pharmaceutically acceptable salt thereof, characterized in that a compound of structural formula:

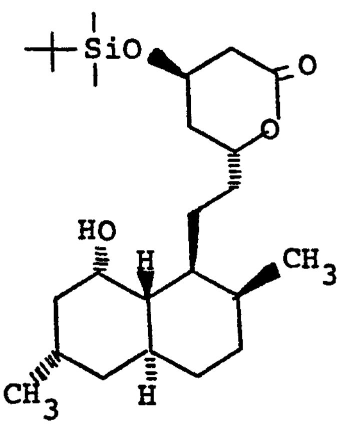

is treated with 2,2-dimethylbutyryl chloride to produce the compound of structural formula:

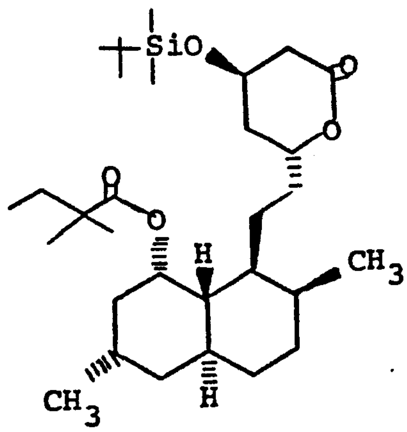

followed by treatment with tetrabutylamonniuim fluoride trihydrate and acetic acid in an ethereal solvent to produce the compound of structural formula I, followed if desired by conversion to a pharmaceutically acceptable salt of compound Ia, or to methyl, ethyl or α-monoglyceride ester.

2. The compound of structural formula I or Ia:

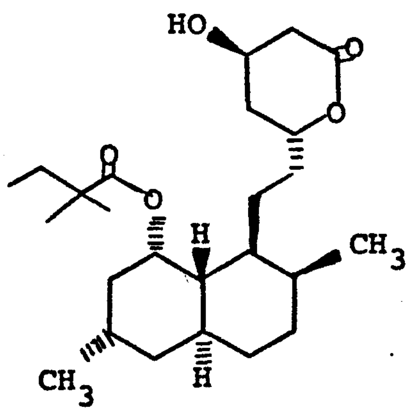

I

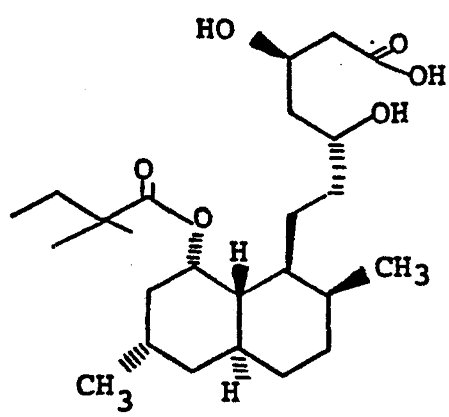

Ia

or a pharmaceutically acceptable salt of Ia, or a methyl, ethyl or α-monoglyceride ester of Ia.

3. A pharmaceutical composition comprising a pharmaceutical carrier and an effective amount of a compound of structural formula I or Ia:

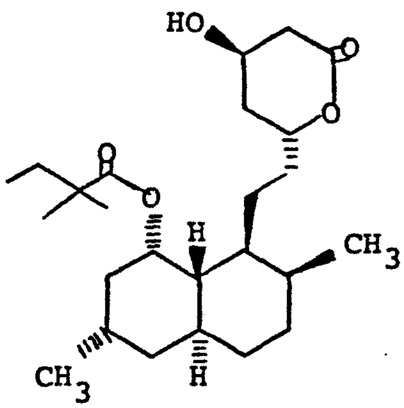

I

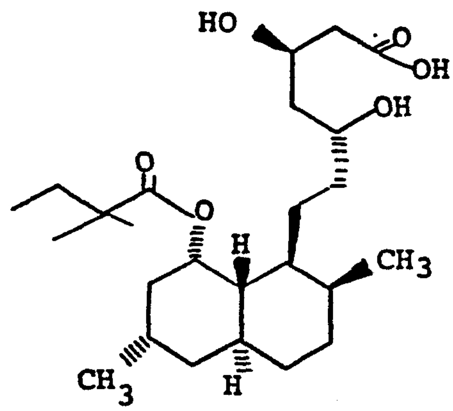

Ia

or a pharmaceutically acceptable salt of Ia, or a methyl, ethyl or α-monoglyceride ester of Ia.

CLAIM FOR AUSTRIA

A process for the preparation of a compound of structural formula I or Ia:

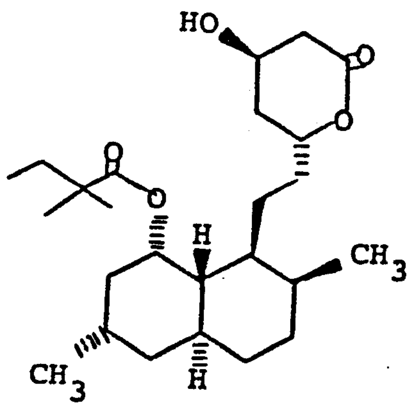

I

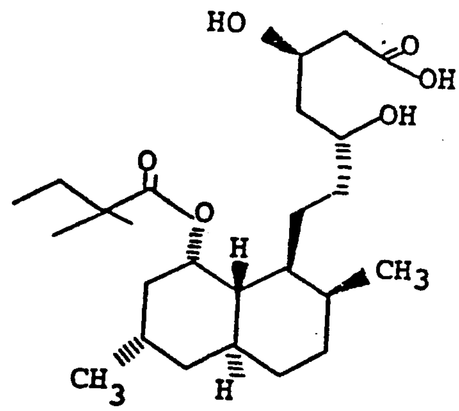

Ia .

or a pharmaceutically acceptable salt thereof, characterized in that a compound of structural formula:

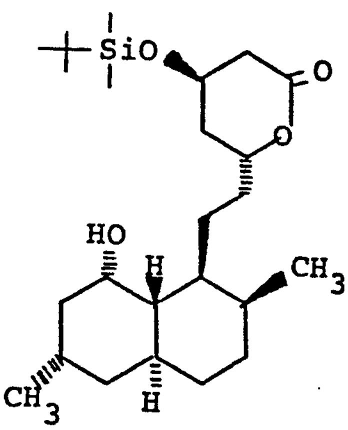

is treated with 2,2-dimethylbutyryl chloride to produce the compound of structural formula:

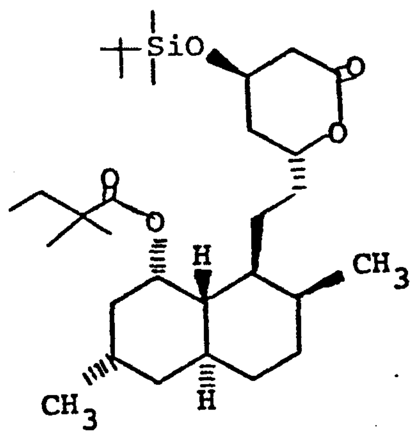

followed by treatment with tetrabutylamonniuim fluoride trihydrate and acetic acid in an ethereal solvent to produce the compound of structural formula I, followed if desired by conversion to a pharmaceutically acceptable salt of compound Ia, or to methyl, ethyl or α-monoglyceride ester.

European Patent Office

# EUROPEAN SEARCH REPORT

0094443
Application number

EP 82 10 4290

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | C 07 D 309/30<br>C 07 C 69/30<br>C 07 C 69/33<br>A 61 K 31/22<br>A 61 K 31/365 |
| A | EP-A-0 033 538 (MERCK)<br>*Pages 1-3,23, Table I; pages 24-27,45-49; examples 4,5; claims*<br>----- | 1-3 | |
| | | | TECHNICAL FIELDS SEARCHED (Int. Cl. 3) |
| | | | C 07 D 309/00<br>C 07 C 69/00 |

The present search report has been drawn up for all claims

| Place of search | Date of completion of the search | Examiner |
|---|---|---|
| THE HAGUE | 19-01-1983 | FRANCOIS J.C.L. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503. 03.82